# EUROPEAN PATENT APPLICATION

(11) **EP 0 925 793 A2**
(43) Date of publication of application: **30.06.1999**
(21) Application number: 98124060.9
(22) Date of filing: 17.12.1998
(51) Int. Cl.: A61L 2/08

(54) **Portable sterilizing apparatus**

(30) Priority: 25.12.1997 JP 36628797
(71) Applicant: Kabushiki Kaisha Lucent, Tokyo (JP)
(72) Inventor: Sakurai, Yoshihiro, C/O Kabushiki Kaisha Lucent, Tokyo (JP)
(74) Representative: Riederer Freiherr von Paar zu Schönau, Anton

(57) **Abstract**

A portable sterilizing apparatus comprises: a portable body housing (1) having a generally part-cylindrical side wall portion; a horizontal bottom floor surface; a flange portion (3) formed to extend parallel with the bottom surface; and an irradiating unit (2) having a shade member (15), a sterilizing lamp (19) with a terminal electrode, an electric socket (20) for receiving the terminal electrode, and a lamp holder (17) provided in the irradiating unit for replaceably supporting the sterilizing lamp. The irradiating unit is pivotally supported on a top portion of the body housing (1) for rotating upward from a front surface (4) of the housing, said body housing being provided at its inside with power source means (10) and means (7) for controlling the conduction of electric power from the power source. The control means (7) include an automatic switch (11) for turning OFF the electric power supplied to the irradiating unit when the irradiating unit assumes a closed position, a timer (13) for pre-limiting the lighting time of the sterilizing lamp (19) when the irradiating unit assumes an open position, a manual switch (14) for turning ON the sterilizing lamp again by external operation after expiration of the lighting time of the sterilizing lamp limited by the timer and a lighting lamp (12) indicating the lighting state of the sterilizing lamp.

## Description

### FIELD OF THE INVENTION

The present invention relates to a portable sterilizing apparatus for sterilizing and disinfecting an implement used in the filed of medical treatment or in the field of barbers' shops and beauty treatment or food or the like, and more specifically to a portable sterilizing apparatus capable of carrying a body thereof to install at a suitable place at a suitable time and easily sterilizing articles to be sterilized such as an implement used as stated above.

### BACKGROUND OF THE INVENTION

In the past, where such an implement or article was sterilized and disinfected by a sterilizing lamp such as a lamp irradiating ultraviolet rays or the like, sterilization and disinfection have been accomplished by moving the article to be sterilized to a sterilizing apparatus. The sterilizing apparatus of this kind is in the form of a large and immovable box, and the article to be sterilized was sterilized while being housed in a sterilizing chamber of the apparatus. Therefore, it has been necessary to move the article to be sterilized to the sterilizing apparatus every time sterilization and disinfection were to take place.

In view of the foregoing, a sterilizing apparatus has been proposed which can be moved by casters as disclosed in Japanese Patent Application Laid-Open NO. 6-154300 Publication. In this sterilizing apparatus, a holder provided on a doctor table is inserted into the body provided with a sterilizing lamp, and the sterilizing lamp is irradiated in a medical implement mounted on the holder. In the sterilizing apparatus as described, it is necessary to change the size of a housing portion of an article to be sterilizing article in which the sterilizing lamp is irradiated adjusting to the size of the article to be sterilized subjected to sterilization and disinfection, which apparatus becomes very large depending on the size of the article to be sterilized.

Further a stand type sterilizing apparatus as disclosed in Japanese registered Utility Model Publication No. 3012680 has been proposed. In this sterilizing apparatus, a sterilizing lamp is mounted on a stand-type box and is transferred to locations required to be sterilized such as a hotel, a restaurant and the like, where a place to be sterilized is sterilized by the sterilizing lamp. Since in the sterilizing apparatus, the sterilizing lamp is merely mounted on the stand-type box, the sterilizing lamp possibly receives an external shock when moving, thus requiring to pay attention. Further in the sterilizing apparatus, when the presence of a person is sensed by a sensor the irradiation of the sterilizing lamp stops, and thus it may be suitable for sterilizing and disinfecting a hotel, a restaurant or the like where no person is present, but not for hospitals or barbers' and beauty shops where persons move in and out frequently.

### SUMMARY OF THE INVENTION

It is an object of the present invention to provide a portable sterilizing apparatus capable of being moved to a place where an implement used in the filed of medical treatment and in the field of barbers' and beauty treatment or food or the like (hereinafter referred to as an article to be sterilized) is present without moving the article to be sterilized to the sterilizing apparatus, and capable of making a sterilizing lamp irradiate to sterilize and disinfect the article to be sterilized in a state of carrying it by hands.

Preferably, the portable sterilizing apparatus should be capable of being moved without having a sterilizing lamp subjected to a shock due to external substances, and of overcoming the an influence on a human body, and which can sterilize and disinfect safely an article to be sterilized even in barbers' and beauty rooms, food stores and the like where people move in and out frequently, and even in homes.

### BRIEF DESCRIPTION OF THE DRAWINGS

FIG. 1 is a side view of the entity of a portable sterilizing apparatus according to the present invention in the state of non-use;
FIG. 2 is a bottom view of the apparatus;
FIG. 3 is a front view of the entire apparatus including a partly cut side view of it;
FIG. 4 is a vertical sectional side view of the apparatus in the state of use;
FIG. 5 is a back view of the apparatus in the state of use; and
FIG. 6 is a sectional view taken in a plane VI-VI in FIG. 3 through an irradiation unit of the portable sterilizing apparatus.

### DESCRIPTION OF THE PREFERRED EMBODIMENT

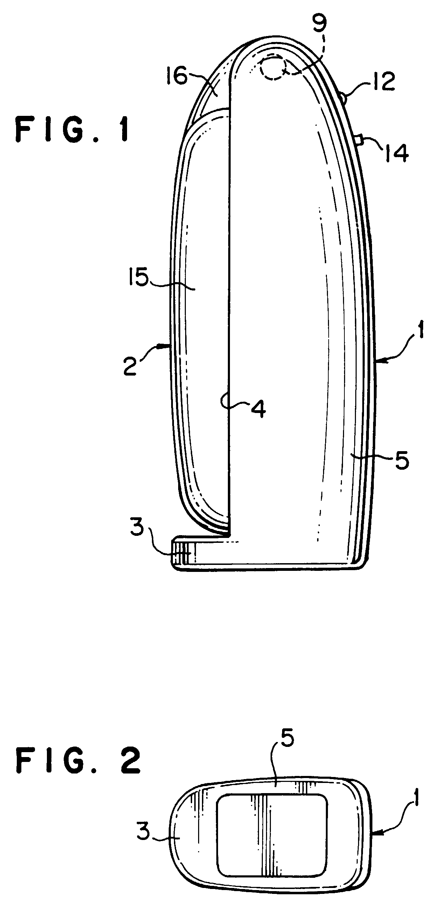

The portable sterilizing apparatus according to the present invention will be explained hereinafter with reference to the drawings. Referring to FIG. 1, a portable sterilizing apparatus comprises a body housing 1 and an irradiating unit 2 rotatably mounted on the housing to be rotated upward from a position in contact with the housing.

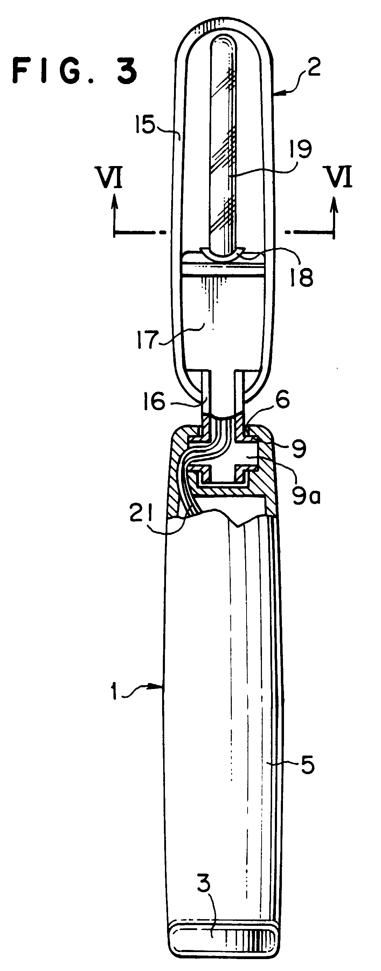

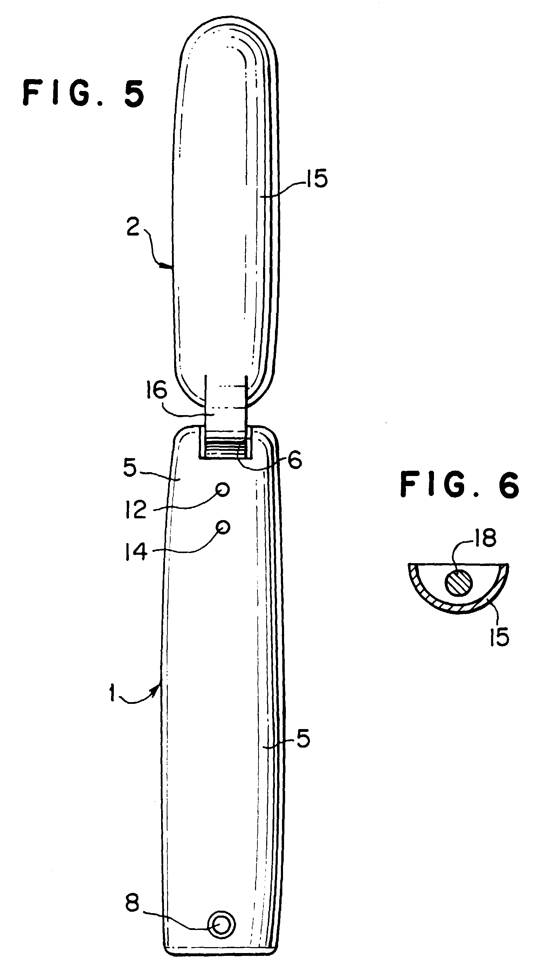

The body housing 1 of the portable sterilizing apparatus comprises, as shown in FIGS. 1 and 2, a bottom surface horizontally parallel to a floor surface associated to a flange portion 3, a top generally in the form of a circular arc, a front surface 4 extending vertically towards the bottom, a rear surface extending generally vertically from the bottom surface opposite to the front surface 4, a height-wise intermediate portion thereof having a most swelling portion to be carried easily, and being curved generally from the intermediate portion towards the top and extending to the top, and a box 5 in a closed hollow state in which extreme ends of both sides are converged generally in the form of circular arcs, and the longitudinal width is wider than the lateral width. The flange portion 3 is bent in a laterally inverted C-shape at the lower part of the front surface 4 and extends parallel with the bottom surface. Further as shown in FIGS. 3 and 5, the body housing 1 of the portable sterilizing apparatus is provided at the upper end with a mounting groove 6 open longitudinally and upwardly at a central position of the lateral width.

The body housing 1 of the portable sterilizing apparatus constituted as described above is preferably of a size to be portable such that the height is approximately 130 mm, the longitudinal width of the bottom surface is approximately 40 mm, and the lateral width is approximately 28 mm.

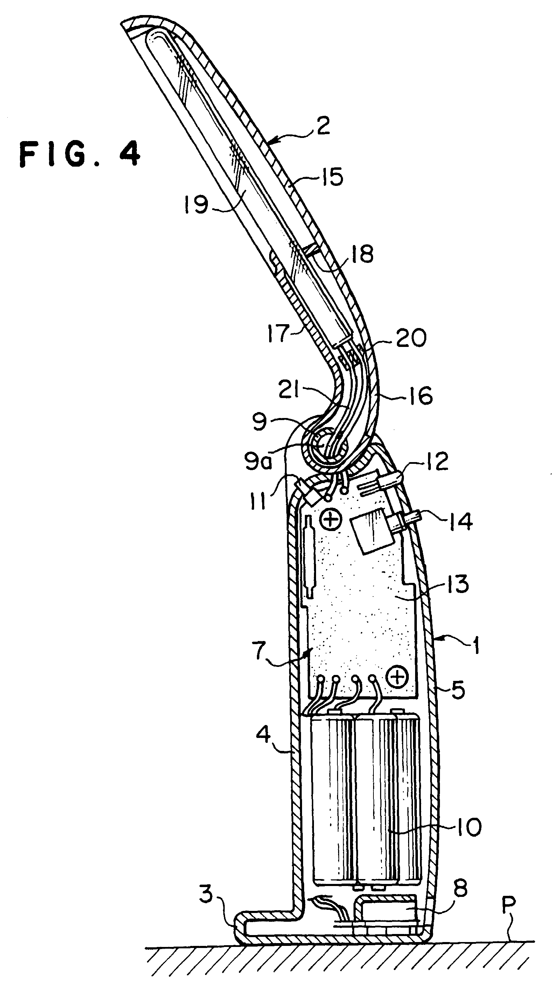

As shown in FIG. 4, the body housing 1 comprises electric switching control means 7 and has a bracket 8 connected to an external power source, and a power source portion 10 such as a storage battery charged by said power source. The control means 7 for controlling the electric conduction include an automatic switch 11 for turning OFF the power supplied to the irradiating unit 2 when the latter assumes a closed position, a lighting display lamp 12 for monitoring, a timer circuit 13 for limiting the supply time of power to be supplied to the irradiating unit, and a manual switch 14 for restarting the timer circuit 13 by an external operation. It is noted that the power source portion 10 can be replaced by a dry battery instead of the storage battery.

The irradiating unit 2 has a shade member 15 which is long in the longitudinal direction of the apparatus and has a longitudinal end portion bent and closed in the form of a semicircular arc as shown in FIG. 3, and which has semicircle section and a hollow form cut in half as shown in FIG. 6, and a mounting arm 16 bent in a curved shape and formed integrally generally at a rear central central position of the shade member 15, the mounting arm 16 being formed with a shaft portion 9 projecting on both sides of the rear end thereof.

Further as shown in FIGS. 3 and 4, the irradiating unit 2 is provided with a lamp holder 17 and with a holding portion 18 for replaceably supporting a sterilizing lamp 19 for irradiating an article to be sterilized, the lamp holder 17 being detachably mounted at the irradiating unit 2.

In the sterilizing lamp supported on the lamp holder 17, the lamp 19 is a small low-voltage mercury lamp of a cold-cathode type covered by ordinarily manufactured quartz glass. It is used with a peak value in emission wavelengths of 185 nm and 254 nm and with a power output of 3 W to 5 W. An appropriate size thereof is approximately 64 to 66 cm in the length and 6.0 to 6.6 in the diameter width.

The holding portion 18 of the irradiating unit 2 serves for holding the rear portion of the lamp 19 at the inner surface in contact with the front end of the lamp holder 17. The lamp 19 held on the holding portion 18 is connected to a terminal electrode of an electric socket 20 on the inner surface positioned at the rear of the front end of the lamp holder 17. The electric socket 20 is connected to a lead wire 21 inserted into a center hole 9a bored in the center of the shaft portion 9 of the mounting arm 16 and connected to a timer circuit through the lead wire 21.

The mounting arm 16 of the irradiating unit 2 is fitted in the mounting groove 6 provided in the top portion of the body housing 1 and is pivotally supported on the top portion of the body housing 1 round the shaft portion 9.

The irradiating unit 2 constituted as described above is normally folded in a state in contact with the front surface 4 of the body housing 1 when the portable sterilizing apparatus is not used. The longitudinal length of the irradiating unit 2 is such that when the former is folded to the body housing 1 opposedly in orientation and in contact therewith, the front end comes closely into contact with the flange portion 3 of the body housing 1. Further when the irradiating unit 2 is closed, the shade member 15 comes in contact with the front surface 4 of the body housing 1, so that the interior of member 15 containing the sterilizing lamp 19 is closed, and the side of the shade member 15 is fit in a longitudinal length with the flange portion 3.

Accordingly, when the portable sterilizing apparatus is not in use, the irradiating unit 2 is folded, and the upper surface of the shade member 15 comes into contact with the vertical surface of the body 1. That is, when the portable sterilizing apparatus is not used, it can completely be held in both arms and carried.

In use, the apparatus is, for example, stood upright on the surface P of a table, and the irradiating unit 2 is turned upward from the body 1 and adjusted to a position opposite to an article to be sterilized. At that time, the irradiating unit 2 can be rotated fractionally with respect to the body 1 and held in position at a suitable angle. The range of angle at which the irradiating unit can be rotated is from 0 degrees to about 150 degrees.

When the irradiating unit 2 is rotated upward, the automatic switch 11 of the means 7 for controlling electric conductance is automatically closed to light the sterilizing lamp 19. When the sterilizing lamp 19 is lighted, the lighting display lamp 12 also emits light.

Accordingly, the irradiating unit 2 is rotated upward to make the sterilizing unit 19 irradiate whereby the article to be sterilized is disinfected and sterilized, and the lighting lamp 12 is turned on. As a result, a user engaged in disinfection and sterilization can pay attention so as not to look straight at the front surface of the irradiating unit 2.

The lighting time of the sterilizing lamp 19 is preset to and for 30 seconds by the timer circuit 13. That is, when the time of the timer circuit 13 has passed 30 seconds, the sterilizing lamp 19 is put out due to being controlled by the timer circuit 13.

Further when the irradiating unit 2 is rotated downward and folded as described above, even in the midst of the passage of 30 minutes of the timer circuit 30, the automatic switch 11 is opened so that the sterilizing lamp 19 is turned OFF, and the energizing state is discontinued.

The lighting time of the sterilizing lamp is set in accordance with the results of testing the sterilizing effect by ZAIDONHOJIN (Foundational Juridical Person) Japanese Food Analyzing Center. Test germs used in the test are NB culture medium of EIKEN CHEMICAL K. K.: ordinary Bouillon culture medium and SA culture medium: Escherichia coli IFO 3301 (coliform bacilli) cultured by standard agar. This test germ was prepared by being inoculated in the NB culture medium, being cultured for 20 hours at 35° C, and having the obtained culture liquid diluted by sterilization refining water so as to have the number of germs result in approximately 105/ml to provide a germ liquid.

In the testing operation, 300 ml of germ liquid were put into a 300 ml glass beaker (diameter: approximately 75 mm). A lamp portion of the sterilizing lamp as an examining portion was inserted into the center portion of the glass beaker and the sterilizing lamp irradiated the germ liquid for 10, 20 and 30 seconds. In the measuring process, the number of raw germs in the germ liquid before irradiation and the number of raw germs in the germ liquid after irradiation for 10, 20 and 30 seconds were measured by a mixing flat-plate culturing process (culturing for two days at 35° C) using the SA culture medium.

As the result of measurement, the measured values as given in the following Table 1 were obtained. As will be apparent from the test results shown in the Table, in the germ liquid subjected to irradiation for 30 seconds, the number of raw germs was reduced most effectively.

**TABLE 1**

| Irradiation time | Number of raw germs per 1 ml |
|---|---|
| 0 (before irradiation) | 7.9 x 10⁶ |
| 10 seconds | 1.5 x 10⁵ |
| 20 seconds | 1.2 x 10⁴ |
| 30 seconds | 3.3 x 10² |

Incidentally, in the portable sterilizing apparatus according to the present invention, in case the sterilization of the irradiated surface of an article to be sterilized by the sterilizing lamp 19 is resultless even though the lighting time of 30 seconds preset by the timer circuit 13 has passed, the sterilizing lamp 19 can be turned on again by depressing the manual switch 14 provided on the body housing 1.

Further in the body housing 1 of the apparatus, since the rear surface thereof has a swell so as to be carried easily and the sterilizing lamp is mounted in the irradiating unit 2 as previously mentioned, where the irradiating surface of an article to be sterilized is wide, a user may rotate the irradiating unit 2 upward to oppose the sterilizing lamp 19 to the article to be sterilized, move the body housing 1 in the state of being carried by hands in a lateral direction along the surface of the article to be sterilized, and sterilize and disinfect the article to be sterilized by the repetition of opening and closing operation of the irradiating unit 2 or operation of the manual switch 14.

As described above, in the portable sterilizing apparatus according to the present invention, the rear surface of the body housing 1 has a swell so as to be carried easily.

Further in the portable sterilizing apparatus according to the present invention, the irradiating unit 2 is folded to the front surface 4 of the body 1 whereby the inner surface of the shade member 15 is closed by the inner or front surface 4 of the body housing 1, and when moving, the sterilizing lamp 19 can be protected safely from a shock possibly caused by foreign substances.

In the sterilizing lamp 19 of the portable sterilizing apparatus according to the present invention, since its lighting state can be known from the lighting display lamp 12, it is possible to avoid the user's erroneously looking straight at the sterilizing lamp 19.

Further since the lighting time of the sterilizing lamp 19 is preset by the timer circuit 13, even if the user is engaged in other work at a distant place, he can sterilize and disinfect the article to be sterilized safely.

In addition, the sterilizing time of the sterilizing lamp 19 can be extended depending on the sterilizing state of the article to be sterilized, but since the time to be extended is limited to a preset time, it is possible to avoid the user's mistaking or forgetting the setting termination time.

## Claims

1. A portable sterilizing apparatus, characterized by:
a. a body housing (1) having a part-cylindrical side wall portion extending lengthwise that can be gripped by hands, and a bottom portion and a top portion defining ends opposite to each other of said side wall portion;
b. an irradiating unit (2) pivotally supported on the top portion of said housing (1), comprising a sterilizing lamp (19) having a terminal electrode, a shade member (15) including a lamp holder (17) for replaceable supporting said sterilizing lamp, and an electric socket (20) that can receive the terminal electrode of said sterilizing lamp;
c. power source means (10) arranged in said body housing (1); and
d. means (7) for supplying electric power to said sterilizing lamp (19) through said electric socket (20) from said power source means (10) and controlling said electric conduction, said means (7) including an operation switch provided outside of the body housing.

2. The portable sterilizing apparatus according to claim 1, characterized in that said irradiating unit (2) has a folded closed position in contact with the front surface (4) of the side wall portion of said body housing (1) and an open position extending in a direction of an axis generally in said side wall portion.

3. The portable sterilizing apparatus according to claim 2, characterized in that the front surface of the side wall portion of said body housing (1) has a surface shape fitted to said irradiating unit (2) in said closed position of the irradiating unit.

4. The portable sterilizing apparatus according to claim 2 or 3, characterized in that said means (7) for controlling the electric conduction includes an automatic switch for turning OFF the electric power supplied to said irradiating unit (2) when said irradiating unit assumes said closed position and a timer for limiting the supply time of the electric power supplied to said irradiating unit when said irradiating unit assumes said open position.

5. The portable sterilizing apparatus according to any of claims 1 to 4, characterized in that the bottom portion of said body housing (1) has a flange portion (3) extended in a lateral direction at an angle from said side wall portion, and said portable sterilizing apparatus can be stood upright when said bottom portion is placed on a horizontal support surface (P).

6. The portable sterilizing apparatus according to any of claims 1 to 5, characterized in that said sterilizing lamp (19) is a small low-voltage mercury lamp of cold cathode type covered by quartz glass, and has an electric consumption of 3 W to 5 W and emitting wavelength of 185 nm and 254 nm at peak time.
